# FASCICULE DE BREVET EUROPEEN

(11) **EP 0 861 835 B1**
(45) Date de publication et mention de la délivrance du brevet: **17.04.2002**
(21) Numéro de dépôt: 98400216.2
(22) Date de dépôt: 03.02.1998
(51) Int. Cl.: C07D 307/79, A61K 7/13, C07D 333/54, C07D 235/06, C07D 235/08, C07D 249/18, C07D 209/08, C07D 209/12, C07D 275/04, C07D 285/14, C07C 215/86

(54) **Compositions pour la teinture des fibres kératiniques contenant des para-aminophénols, procédé de teinture, nouveaux para-aminophénols et leur procédé de préparation**
Para-Aminophenole enthaltende Zubereitungen zur Färbung von Keratinfasern, Färbeverfahren, Para-Aminophenole und ein Verfahren zu deren Herstellung
Para-aminophenols containing compositions for dyeing of keratin fibres, dyeing process, para-aminophenols and a method for their preparation

(30) Priorité: 26.02.1997 FR 9702307
(43) Date de publication de la demande: 02.09.1998
(73) Titulaire: L'OREAL, 75008 Paris (FR)
(72) Inventeur: Vandenbossche, Jean-Jacques, 40400 Begaar (FR); Lagrange, Alain, 77770 Coupvray (FR)
(74) Mandataire: Goulard, Sophie

(56) Documents cités:
- EP-A- 0 428 441
- WO-A-92/17157
- DE-A- 2 816 785
- DE-B- 2 704 793
- PATENT ABSTRACTS OF JAPAN vol. 013, no. 011 (C-558), 11 janvier 1989 & JP 63 216861 A (NIPPON KAYAKU CO LTD), 9 septembre 1988
- G. MENICHI ET AL.: "No. 416. - Recherches sur le benzofuranne. LIII. - Possibilité d'accès aux furobenzoxazoles par l'intermédiaire d'arylbenzofurannes ortho hydroxylés" BULLETIN DE LA SOCIETE CHIMIQUE DE FRANCE., no. 7-8, 1973, PARIS FR, pages 2352-2354, XP002045612
- CHEMICAL ABSTRACTS, vol. 79, no. 19, 12 novembre 1973 Columbus, Ohio, US; abstract no. 115501, I.A. BELENKAYA ET AL.: "Oxidation and reduction of some benzo-2,1,3-thiadiazole derivatives" XP002045613 & KHIM. GETEROTSIKL. SOEDIN., no. 7, 1973, pages 926-929
- CHEMICAL ABSTRACTS, vol. 103, no. 25, 23 décembre 1985 Columbus, Ohio, US; abstract no. 208461d, F.U. ERKOG ET AL.: "Metabolism of trifluralin in rats" page 305; colonne 2; XP002045614 & J. AGRIC. FOOD CHEM., vol. 33, no. 6, 1985, pages 1061-1070
- CHEMICAL ABSTRACTS, vol. 82, no. 7, 17 février 1975 Columbus, Ohio, US; abstract no. 38688, V.G. VLADIMIROV ET AL.: "Radioprotective activity of some 5-methylbenzo-2,1,3-thiadiazole derivatives" XP002045615 & RADIOBIOLOGIYA, vol. 14, no. 4, 1974, pages 607-610

## Description

L'invention a pour objet de nouvelles compositions pour la teinture d'oxydation des fibres kératiniques comprenant au moins un para-aminophénol à titre de base d'oxydation, le procédé de teinture mettant en oeuvre cette composition, de nouveaux para-aminophénols ainsi que leur procédé de préparation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tel que par exemple les aminoindoles décrits dans la demande de brevet WO-A-92/17157.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La demanderesse vient maintenant de découvrir, de façon totalement inattendue et surprenante que certains para-aminophénols de formule (I) définie ci-après, pour partie nouveaux en soi, conviennent pour une utilisation comme précurseurs de colorant d'oxydation.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture, à titre de base d'oxydation, au moins un para-aminophénol de formule (I) suivante, et/ou au moins l'un de leurs sels d'addition avec un acide : 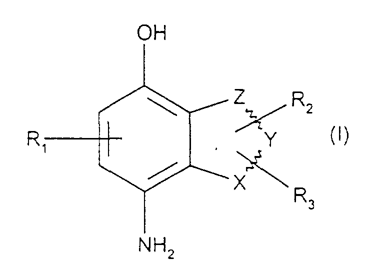 dans laquelle :
- R₁ représente un atome d'hydrogène ; un radical alkyle en C₁-C₄ linéaire ou ramifié ou un radical trifluoroalkyle en C₁-C₄,
- R₂ et R₃, identiques ou différents, représentent un atome d'hydrogène, un radical alkyle en C₁-C₄ ou un radical monohydroxyalkyle en C₁-C₄,
- X, Y et Z, identiques ou différents, représentent un atome de carbone, d'azote, d'oxygène ou de soufre,
- les liaisons XY et YZ peuvent être simples ou doubles,

Comme indiqué précédemment, les compositions de teinture d'oxydation conformes à l'invention permettent de conduire à une coloration des fibres kératiniques en milieu oxydant.

Un autre objet de l'invention est l'utilisation des para-aminophénols de formule (I) à titre de base d'oxydation dans des compositions pour la teinture d'oxydation des fibres kératiniques et en particulier des fibres kératiniques humaines telles que les cheveux.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Parmi les para-aminophénols de formule (I), utilisables à titre de base d'oxydation dans les compositions conformes à l'invention, on peut notamment citer :
- le 4-amino-2,2-diméthyl-2,3-dihydro-benzofuran-7-ol,
- le 7-amino-3H-benzoimidazol-4-ol,
- le 7-amino-benzo[b]thiophen-4-ol,
- le 7-amino-2,3-diméthyl-benzofuran-4-ol,
- le 4-amino-2,3-diméthyl-benzofuran-7-ol,
- le 7-amino-1-propyl-5-trifluoromethyl-1H-benzoimidazol-4-ol,
- le 7-amino-benzo[b]isothiazol-4-ol,
- le 7-amino-1H-benzotriazol-4-ol,
- le 4-amino-2,3-dihydro-1H-indol-7-ol,
- le 7-amino-indan-4-ol,
- le 4-amino-5-méthyl-2,1,3-benzothiadiazol-4-ol,
- le 7-amino-2,1,3-benzothiadiazol-4-ol,
- le 7-amino-1-méthyl-1H-indol-4-ol,
- le 7-amino-1-(2-hydroxyéthyl)-1H-indol-4-ol,
et leurs sels d'addition avec un acide.

Parmi ces para-aminophénols, on préfère plus particulièrement :
- le 4-amino-2,2-diméthyl-2,3-dihydro-benzofuran-7-ol,
- le 7-amino-3H-benzoimidazol-4-ol,
- le 7-amino-1-méthyl-1H-indol-4-ol,
et leurs sels d'addition avec un acide.

Le ou les para-aminophénols de formule (I) ci-dessus représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (Il) suivante : 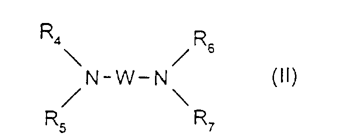 dans laquelle W est un reste propylène éventuellement substitué par un groupement hydroxyle ou un radical alkyle en C₁-C₄ ; R₄, R₅, R₆ et R₇, identiques ou différents, représentent un atome d'hydrogène, un radical alkyle en C₁-C₄ ou hydroxyalkyle en C₁-C₄.

La composition tinctoriale conforme à l'invention peut encore contenir, en plus des colorants de formule (I) définie ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques différentes des para-aminophénols de formule (I) utilisés conformément à l'invention.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR-A-2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols, on peut plus particulièrement citer à titre d'exemple, citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques, les dérivés pyrazoliques, et leurs sels d'addition avec un acide.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Certains para-aminophénols de formule (I), utilisés à titre de base d'oxydation dans le cadre de la présente invention, sont nouveaux et, à ce titre, constituent un autre objet de l'invention.

Ces nouveaux para-aminophénols sont :
- le 4-amino-2,2-diméthyl-2,3-dihydro-benzofuran-7-ol,
- le 7-amino-1-méthyl-1H-indol-4-ol,
et leurs sels d'addition avec un acide.

Les para-aminophénols de formule (I) conformes à l'invention peuvent par exemple être préparés selon le procédé général répondant au schéma de synthèse suivant : 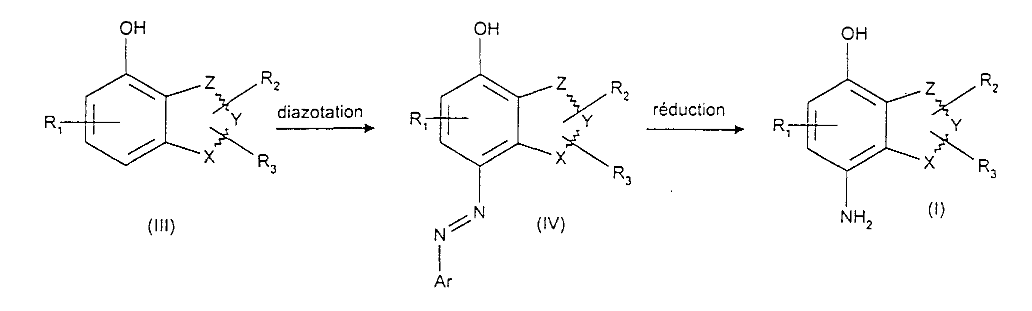 consistant dans une première étape, à alcaliniser une solution aqueuse d'un phénol de formule (III) dans laquelle les radicaux R₁, R₂ et R₃, ainsi que les atomes X, Y et Z ont les mêmes significations que celles indiquées précédemment pour la formule (I), à l'aide d'une base forte, à une température comprise entre -15°C et 20°C et de préférence entre -5°C et 5°C, pour obtenir une solution aqueuse du phénate correspondant, puis dans une deuxième étape à faire réagir le phénate obtenu à la première étape avec un sel de diazonium d'une amine aromatique en maintenant la température du milieu entre -15°C et 20°C, et de préférence entre - 5°C et 5°C, pour obtenir le composé diazonium de formule (IV) correspondant dans lequel les radicaux R₁, R₂ et R₃, ainsi que les atomes X, Y et Z ont les mêmes significations que celles indiquées précédemment pour la formule (I), puis, dans une troisième étape, à réduire le composé diazonium de formule (IV), soit par réduction chimique, soit par réduction par hydrogénation catalytique en présence d'un catalyseur d'oxydation et d'hydrogène ou d'un donneur d'hydrogène, pour obtenir le para-aminophénol de formule (I) correspondant.

L'agent alcalinisant utilisé lors de la première étape est de préférence choisi parmi les bases fortes telles que la soude et la potasse.

L'amine aromatique utilisée lors de la deuxième étape est de préférence choisie parmi les anilines non substituées ou mono- ou polysubstituées par un radical alkyle en C₁-C₄, alcoxy en C₁-C₄, dialkylamino en C₁-C₄, nitro, carboxyle ou sulfonyle.

A titre d'amine aromatique, on préfère tout particulièrement utiliser l'aniline ou l'acide sulfanilique.

Le sel de diazonium de l'amine aromatique utilisé lors de la deuxième étape, est de préférence réalisé par dissolution de l'amine aromatique dans une solution d'acide fort tel que par exemple l'acide chlorhydrique, dans laquelle on ajoute ensuite une solution de nitrite de sodium en maintenant la température du milieu entre -15°C et 20°C et de préférence entre -5°C et 5°C.

Lorsque la réduction du composé de formule (IV) est réalisée par réduction chimique on utilise de préférence, à titre d'agent réducteur, du dithionite, du fer en milieu acétique ou du zinc en milieu alcoolique.

Lorsque la réduction du composé de formule (IV) est réalisée par hydrogénation catalytique, l'hydrogène utilisé selon le procédé de l'invention est de préférence de l'hydrogène moléculaire sous faible pression.

Parmi les donneurs d'hydrogène, on peut citer l'acide formique et les donneurs d'hydrogène oléfiniques tels que par exemple le cyclohexène, les cyclohexènes substitués, le 1,3-cyclohexadiène et le 1,4-cyclohexadiène.

A titre de catalyseur d'hydrogénation, on peut par exemple citer les métaux choisis dans le groupe constitué par le chrome, le molybdène, le tungstène, le platine, le palladium, le rhodium, le cobalt, le nickel et le ruthénium, leurs oxydes, et les combinaisons de ces substances, telles que par exemple un mélange d'oxyde de cobalt et de molybdène y compris le molybdate de cobalt.

Les catalyseurs d'hydrogénation préférés sont le palladium ou le nickel de Raney, de même que d'autres métaux du groupe du platine. De façon connue, le catalyseur peut être déposé sur un support inerte de pH neutre de façon à ne pas influencer le pH du milieu solvant réactionnel. Parmi ces supports inertes, on peut citer par exemple le charbon de bois neutre, le charbon neutre, l'alumine neutre, les zéolithes, les argiles, etc. On utilise de préférence du charbon neutre.

Les catalyseurs d'hydrogénation sont en général présents en une quantité comprise entre 0,2 et 5 % en poids d'équivalent métal par rapport au poids du composé de formule (IV) à faire réagir.

Lorsque la réaction est terminée, les para-aminophénols de formule (I) conformes à l'invention peuvent, le cas échéant, être récupérés par des méthodes bien connues de l'état de la technique telles que la cristallisation, la distillation ou l'entraînement à la vapeur.

Les exemples qui suivent sont destinés à illustrer l'invention sans pour autant en limiter la portée.

### EXEMPLE DE PREPARATION

### EXEMPLE DE PREPARATION 1 : Synthèse du 4-amino-2,2-diméthyl-2,3-dihydro-benzofuran-7-ol

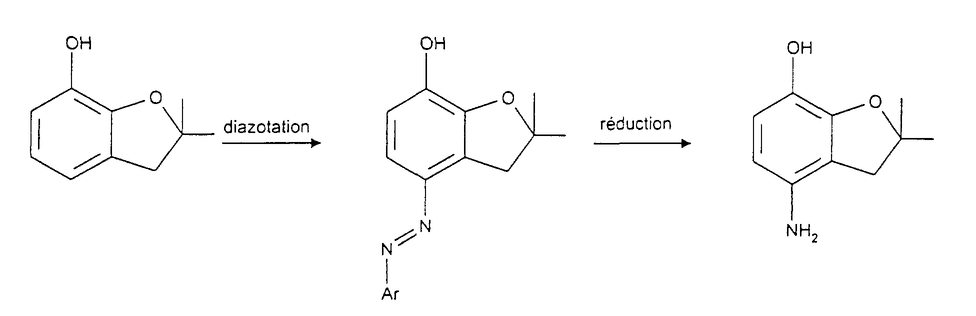

### a) Préparation du 2,2-diméthyl-4-phénylazo-2,3-dihydro-benzofuran-7-ol

On a dissous 56 grammes d'aniline (0,6 mole) dans un mélange de 800 ml d'eau et de glace acidifié par 130 ml d'acide chlorhydrique 12N. On a ensuite coulé en 10 minutes dans ce mélange, 100 ml d'une solution aqueuse de 46 g de nitrite de sodium (0,66 mole), tout en maintenant la température de réaction à 0°C.

La solution jaune pâle ainsi obtenue, a été maintenue à 0°C et coulée en 1 heure, sous agitation, dans une solution qui avait été obtenue par dissolution de 98,4 g de phénol (0,6 mole) dans un mélange de 1500 ml d'eau et de glace alcalinisée par 90 ml de soude 10N en 30 minutes et à 0°C.

On a obtenu un précipité orange qui a été filtré, lavé à l'eau puis séché pour obtenir 154 g de composé azoïque désiré. Le produit obtenu a ensuite été recristallisé du cyclohexane pour conduire à des cristaux oranges dont le point de fusion était de 98°C. L'analyse élémentaire calculée pour C₁₆H₁₆N₂O₂ était :

| **%** | **C** | **H** | **N** | **Cl** |
|---|---|---|---|---|
| Calculé | 71,62 | 6,01 | 10,44 | 11,93 |
| Trouvé | 71,69 | 6,12 | 10,18 | 11,43 |

### b) Préparation du 4-amino-2,2-diméthyl-2,3-dihydro-benzofuran-7-ol

On a mélangé 134 g (0,5 mole) du composé azoïque obtenu ci-dessus à l'étape précédente, avec 540 ml d'alcool à 96° et 268 ml de cyclohexene, puis on a ajouté 30 g de palladium sur charbon à 10% en poids contenant 50 % d'eau. La suspension orange a été portée au reflux. Après 30 minutes la décoloration était complète et on filtré à chaud sur verre fritté. On a refroidi le filtrat ainsi obtenu à 0°C. Le précipité a été récupéré puis filtré, lavé à l'alcool, à l'ether isopropylique, à l'ether de pétrole puis séché. On a obtenu 72 g de produit. Celui a été recristallisé de l'éthanol à 96° et a conduit à des cristaux blancs de 4-amino-2,2-diméthyl-2,3-dihydro-benzofuran-7-ol dont le point de fusion était de 194°C. L'analyse élémentaire pour C₁₀H₁₃NO₂ était :

| % | C | H | N | Cl |
|---|---|---|---|---|
| Calculé | 67,02 | 7,31 | 7,82 | 17,85 |
| Trouvé | 67,24 | 7,42 | 7,92 | 17,72 |

### EXEMPLES D'APPLICATION

### EXEMPLES 2 à 5 DE TEINTURE EN MILIEU ALCALIN

On a préparé les compositions tinctoriales, conformes à l'invention, suivantes (teneurs en grammes) :

| **COMPOSITION** | **2** | **3** | **4** | **5** |
|---|---|---|---|---|
| 4-amino-2,2-diméthyl-2,3-dihydro-benzofuran-7-ol | 0,537 | 0,537 | - | - |
| 7-amino-3H-benzoimidazol-4-ol, chlorhydrate | - | - | 0,627 | 0,637 |
| 3-amino phénol | 0,327 | - | 0,327 | - |
| 5-N-(β-hydroxyéthyl)amino 2-méthyl phénol | - | 0,501 | - | 0,501 |
| Support de teinture commun | (*) | (*) | (*) | (*) |
| Eau déminéralisée q.s.p. | 100 g | 100 g | 100 g | 100 g |

| | | | | |
|---|---|---|---|---|
| (*) support de teinture commun : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN 012 par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Ammoniaque à 20 % de NH₃ 10 g | | | | |

Au moment de l'emploi, on a mélangé chaque composition tinctoriale 2 à 5 avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs ou sur des cheveux permanentés. Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **NUANCE SUR CHEVEUX NATURELS** | **NUANCE SUR CHEVEUX PERMANENTES** |
|---|---|---|
| **2** | légèrement doré | légèrement doré |
| **3** | léger beige irisé | irisé légèrement cuivré |
| **4** | très léger cendré | naturel gris |
| **5** | cendré mat | gris mat |

## Revendications

1. Composition pour la teinture d'oxydation des fibres kératiniques et en particulier des fibres kératiniques et en particulier des fibres kératiniques humaines telles que les cheveux, **caractérisée par le fait qu'**elle comprend, dans un milieu approprié pour la teinture, à titre de base d'oxydation, au moins un para-aminophénol de formule (I) suivante, et/ou au moins l'un de leurs sels d'addition avec un acide : 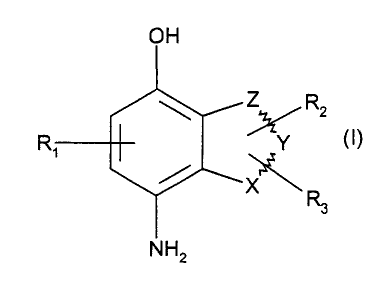 dans laquelle :
- R₁ représente un atome d'hydrogène ; un radical alkyle en C₁-C₄ linéaire ou ramifié ou un radical trifluoroalkyle en C₁-C₄,
- R₂ et R₃, identiques ou différents, représentent un atome d'hydrogène, un radical alkyle en C₁-C₄ ou un radical monohydroxyalkyle en C₁-C₄,
- X, Y et Z, identiques ou différents, représentent un atome de carbone, d'azote, d'oxygène ou de soufre,
- les liaisons XY et YZ peuvent être simples ou doubles,

2. Composition selon la revendication 1, **caractérisée par le fait que** les para-aminophénols de formule (I) sont choisis parmi :
- le 4-amino-2,2-diméthyl-2,3-dihydro-benzofuran-7-ol,
- le 7-amino-3H-benzoimidazol-4-ol,
- le 7-amino-benzo[b]thiophen-4-ol,
- le 7-amino-2,3-diméthyl-benzofuran-4-ol,
- le 4-amino-2,3-diméthyl-benzofuran-7-ol,
- le 7-amino-1-propyl-5-trifluoromethyl-1H-benzoimidazol-4-ol,
- le 7-amino-benzo[b]isothiazol-4-ol,
- le 7-amino-1H-benzotriazol-4-ol,
- le 4-amino-2,3-dihydro-1H-indol-7-ol,
- le 7-amino-indan-4-ol,
- le 4-amino-5-méthyl-2,1,3-benzothiadiazol-4-ol,
- le 7-amino-2,1,3-benzothiadiazol-4-ol,
- le 7-amino-1-méthyl-1H-indol-4-ol,
- le 7-amino-1-(2-hydroxyéthyl)-1H-indol-4-ol,
et leurs sels d'addition avec un acide.

3. Composition selon la revendication 2, **caractérisée par le fait que** les para-aminophénols de formule (I) sont choisis parmi :
- le 4-amino-2,2-diméthyl-2,3-dihydro-benzofuran-7-ol,
- le 7-amino-3H-benzoimidazol-4-ol,
- le 7-amino-1-méthyl-1H-indol-4-ol,
et leurs sels d'addition avec un acide.

4. Composition selon l'une quelconque des revendications précédentes, **caractérisée par le fait que** le ou les para-aminophénols de formule (I) représentent de 0,0005 à 12 % en poids du poids total de la composition tinctoriale.

5. Composition selon la revendication 4, **caractérisée par le fait que** le ou les para-aminophénols de formule (I) représentent de 0,005 à 6 % en poids du poids total de la composition tinctoriale.

6. Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

7. Composition selon l'une quelconque des revendications précédentes, **caractérisée par le fait qu'**elle présente un pH compris entre 3 et 12.

8. Composition selon l'une quelconque des revendications précédentes, **caractérisée par le fait qu'**elle renferme au moins une base d'oxydation additionnelle choisie parmi les paraphénylènediamines, les bis-phénylalkylènediamines, les para-aminophénols, les ortho-aminophénols et les bases hétérocycliques différentes des para-aminophénols de formule (I).

9. Composition selon la revendication 8, **caractérisée par le fait que** la ou les bases d'oxydation additionnelles représentent de 0,0005 à 12 % en poids du poids total de la composition tinctoriale.

10. Composition selon l'une quelconque des revendications précédentes, **caractérisée par le fait qu'**elle renferme au moins un coupleur et/ou au moins un colorant direct.

11. Composition selon la revendication 10, **caractérisée par le fait que** le ou les coupleurs sont choisis parmi les métaphénylènediamines, les méta-aminophénols, les métadiphénols et les coupleurs hétérocycliques, et leurs sels d'addition avec un acide.

12. Composition selon la revendication 10 ou 11, **caractérisée par le fait que** le ou les coupleurs représentent de 0,0001 à 10 % en poids du poids total de la composition tinctoriale.

13. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

14. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 13, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

15. Procédé selon la revendication 14, **caractérisé par le fait que** l'agent oxydant présent dans la composition oxydante est choisi parmi le peroxyde d'hydrogène, le peroxyde d'urée, les bromates de métaux alcalins, les persels tels que les perborates et persulfates.

16. Dispositif à plusieurs compartiments, ou "kit" de teinture à plusieurs compartiments, dont un premier compartiment renferme une composition tinctoriale telle que définie à l'une quelconque des revendications 1 à 13 et un second compartiment renferme une composition oxydante.

17. - le 4-amino-2,2-diméthyl-2,3-dihydro-benzofuran-7-ol,
- le 7-amino-1-méthyl-1H-indol-4-ol,
et leurs sels d'addition avec un acide.

18. Utilisation des para-aminophénols de formule (I) tels que définis à la revendication 1, à titre de base d'oxydation dans des compositions pour la teinture d'oxydation des fibres kératiniques.

## Claims

1. Composition for the oxidation dyeing of keratin fibres, and in particular human keratin fibres such as the hair, **characterized in that** it comprises, in a medium which is suitable for dyeing, as oxidation base, at least one para-aminophenol of formula (I) below, and/or at least one of the addition salts thereof with an acid: 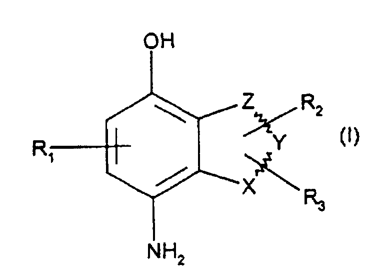 in which:
- R₁ represents a hydrogen atom, a linear or branched C₁-C₄ alkyl radical or a C₁-C₄ trifluoroalkyl radical,
- R₂ and R₃, which may be identical or different, represent a hydrogen atom, a C₁-C₄ alkyl radical or a C₁-C₄ monohydroxyalkyl radical,
- X, Y and Z, which may be identical or different, represent a carbon, nitrogen, oxygen or sulphur atom,
- the bonds XY and YZ can be single or double bonds.

2. Composition according to Claim 1, **characterized in that** the para-aminophenols of formula (I) are chosen from:
- 4-amino-2,2-dimethyl-2,3-dihydrobenzofuran-7-ol,
- 7-amino-3H-benzoimidazol-4-ol,
- 7-aminobenzo[b]thiophen-4-ol,
- 7-amino-2,3-dimethylbenzofuran-4-ol,
- 4-amino-2,3-dimethylbenzofuran-7-ol,
- 7-amino-1-propyl-5-trifluoromethyl-lH-benzoimidazol-4-ol,
- 7-aminobenzo[b]isothiazol-4-ol,
- 7-amino-1H-benzotriazol-4-ol,
- 4-amino-2,3-dihydro-1H-indol-7-ol,
- 7-aminoindan-4-ol,
- 4-amino-5-methyl-2,1,3-benzothiadiazol-4-ol,
- 7-amino-2,1,3-benzothiadiazol-4-ol,
- 7-amino-1-methyl-1H-indol-4-ol,
- 7-amino-1-(2-hydroxyethyl)-1H-indol-4-ol,
and the addition salts thereof with an acid.

3. Composition according to Claim 2, **characterized in that** the para-aminophenols of formula (I) are chosen from:
- 4-amino-2,2-dimethyl-2,3-dihydrobenzofuran-7-ol,
- 7-amino-3H-benzoimidazol-4-ol,
- 7-amino-1-methyl-1H-indol-4-ol,
and the addition salts thereof with an acid.

4. Composition according to any one of the preceding claims, **characterized in that** the para-aminophenol(s) of formula (I) represent(s) from 0.0005 to 12% by weight relative to the total weight of the dye composition.

5. Composition according to Claim 4, **characterized in that** the para-aminophenol(s) of formula (I) represent(s) from 0.005 to 6% by weight relative to the total weight of the dye composition.

6. Composition according to any one of the preceding claims, **characterized in that** the medium which is suitable for dyeing (or the support) consists of water or of a mixture of water and at least one organic solvent chosen from C₁-C₄ lower alkanols, glycerol, glycols and glycol ethers, aromatic alcohols, similar products and mixtures thereof.

7. Composition according to any one of the preceding claims, **characterized in that** it has a pH of between 3 and 12.

8. Composition according to any one of the preceding claims, **characterized in that** it contains at least one additional oxidation base chosen from para-phenylenediamines, bis (phenyl) alkylenediamines, para-aminophenols, ortho-aminophenols and heterocyclic bases other than the para-aminophenols of formula (I).

9. Composition according to Claim 8, **characterized in that** the additional oxidation base(s) represent(s) from 0.0005 to 12% by weight relative to the total weight of the dye composition.

10. Composition according to any one of the preceding claims, **characterized in that** it contains at least one coupler and/or at least one direct dye.

11. Composition according to Claim 10, **characterized in that** the coupler(s) is(are) chosen from meta-phenylenediamines, meta-aminophenols, meta-diphenols and heterocyclic couplers, and the addition salts thereof with an acid.

12. Composition according to Claim 10 or 11, **characterized in that** the coupler(s) represent(s) from 0.0001 to 10% by weight relative to the total weight of the dye composition.

13. Composition according to any one of the preceding claims, **characterized in that** the addition salts with an acid are chosen from the hydrochlorides, hydrobromides, sulphates, tartrates, lactates and acetates.

14. Process for dyeing keratin fibres, and in particular human keratin fibres such as the hair, **characterized in that** at least one dye composition as defined in any one of Claims 1 to 13 is applied to these fibres and the colour is developed at acidic, neutral or alkaline pH using an oxidizing agent which is added to the dye composition only at the time of use, or which is present in an oxidizing composition that is applied simultaneously or sequentially in a separate manner.

15. Process according to Claim 14, **characterized in that** the oxidizing agent present in the oxidizing composition is chosen from hydrogen peroxide, urea peroxide, alkali metal bromates, and persalts such as perborates and persulphates.

16. Multi-compartment device or multi-compartment dyeing "kit", a first compartment of which contains a dye composition as defined in any one of Claims 1 to 13, and a second compartment of which contains an oxidizing composition.

17. 4-Amino-2,2-dimethyl-2,3-dihydrobenzofuran-7-ol,
- 7-amino-1-methyl-lH-indol-4-ol,
and the addition salts thereof with an acid.

18. Use of the para-aminophenols of formula (I) as defined in Claim 1, as oxidation base in compositions for the oxidation dyeing of keratin fibres.

## Patentansprüche

1. Zusammensetzung zum oxidativen Färben von Keratinfasern und insbesondere menschlichen Keratinfasern, wie dem Haar, **dadurch gekennzeichnet, dass** sie in einem zum Färben geeigneten Medium als Oxidationsbase mindestens ein p-Aminophenol der folgenden Formel (I) und/oder mindestens ein Additionssalz dieser Verbindungen mit einer Säure enthält: 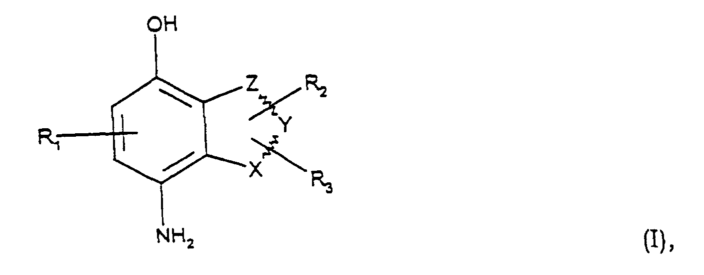 worin:
- R₁ ein Wasserstoffatom, eine geradkettige oder verzweigte C₁₋₄-Alkylgruppe oder eine C₁₋₄-Trifluoralkylgruppe bedeutet,
- die Gruppen R₂ und R₃, die identisch oder voneinander verschieden sind, ein Wasserstoffatom, eine C₁₋₄-Alkylgruppe oder eine C₁₋₄-Monohydroxyalkylgruppe bedeuten,
- X, Y und Z, die identisch oder voneinander verschieden sind, ein Kohlenstoffatom, ein Stickstoffatom, ein Sauerstoffatom oder ein Schwefelatom bedeuten und
- die Bindung zwischen X und Y und die Bindung zwischen Y und Z Einfachbindungen oder Doppelbindungen sein können.

2. Zusammensetzung nach Anspruch 1, **dadurch gekennzeichnet, dass** die p-Aminophenole der Formel (I) ausgewählt sind unter:
- 4-Amino-2,2-dimethyl-2,3-dihydro-benzofuran-7-ol,
- 7-Amino-3*H*-benzimidazol-4-ol,
- 7-Amino-benzo[*b*]thiophen-4-ol,
- 7-Amino-2,3-dimethyl-benzofuran-4-ol,
- 4-Amino-2,3-dimethyl-benzofuran-7-ol,
- 7-Amino-1-propyl-5-trifluormethyl-1*H*-benzimidazol-4-ol,
- 7-Amino-benzo[*b*]isothiazol-4-ol,
- 7-Amino-1*H*-benzotriazol-4-ol,
- 4-Amino-2,3-dihydro-1*H*-indol-7-ol,
- 7-Amino-indan-4-ol,
- 4-Amino-5-methyl-2,1,3-benzothiadiazol-4-ol,
- 7-Amino-2,1,3-benzothiadiazol-4-ol,
- 7-Amino-1-methyl-1*H*-indol-4-ol,
- 7-Amino-1-(2-hydroxyethyl)-1*H*-indol-4-ol
und den Additionssalzen dieser Verbindungen mit einer Säure.

3. Zusammensetzung nach Anspruch 2, **dadurch gekennzeichnet, dass** die p-Aminophenole der Formel (I) ausgewählt sind unter:
- 4-Amino-2,2-dimethyl-2,3-dihydro-benzofuran-7-ol,
- 7-Amino-*3H*-benzimidazol-4-ol,
- 7-Amino-1-methyl-*1H*-indol-4-ol
und den Additionssalzen dieser Verbindungen mit einer Säure.

4. Zusammensetzung nach einem der vorhergehenden Ansprüche, **dadurch gekennzeichnet, dass** das p-Aminophenol oder die p-Aminophenole der Formel (I) 0,0005 bis 12 Gew.-% des Gesamtgewichts der Färbemittelzusammensetzung ausmachen.

5. Zusammensetzung nach Anspruch 4, **dadurch gekennzeichnet, dass** das p-Aminophenol oder die p-Aminophenole der Formel (I) 0,005 bis 6 Gew.-% des Gesamtgewichts der Färbemittelzusammensetzung ausmachen.

6. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösemittel besteht, das unter niederen Alkanolen mit 1 bis 4 Kohlenstoffatomen, Glycerin, Glykolen und Glykolethern, aromatischen Alkoholen, analogen Produkten und deren Gemischen ausgewählt ist.

7. Zusammensetzung nach einem der vorhergehenden Ansprüche, **dadurch gekennzeichnet, dass** sie einen pH-Wert im Bereich von 3 bis 12 aufweist.

8. Zusammensetzung nach einem der vorhergehenden Ansprüche, **dadurch gekennzeichnet, dass** sie mindestens eine zusätzliche Oxidationsbase enthält, die unter p-Phenylendiaminen, Bisphenylalkylendiaminen, p-Aminophenolen, o-Aminophenolen und heterocyclischen Basen, die von den p-Aminophenolen der Formel (I) verschieden sind, ausgewählt ist.

9. Zusammensetzung nach Anspruch 8, **dadurch gekennzeichnet, dass** die zusätzliche Oxidationsbase oder die zusätzlichen Oxidationsbasen 0,0005 bis 12 Gew.-% des Gesamtgewichts der Färbemittelzusammensetzung ausmachen.

10. Zusammensetzung nach einem der vorhergehenden Ansprüche, **dadurch gekennzeichnet, dass** sie mindestens einen Kuppler und/oder mindestens einen Direktfarbstoff enthält.

11. Zusammensetzung nach Anspruch 10, **dadurch gekennzeichnet, dass** der oder die Kuppler unter m-Phenylendiaminen, m-Aminopheolen, 1,3-Dihydroxybenzolen, heterocyclischen Kupplern und den Additionssalzen dieser Verbindungen mit einer Säure ausgewählt sind.

12. Zusammensetzung nach Anspruch 10 oder 11, **dadurch gekennzeichnet, dass** der oder die Kuppler 0,0001 bis 10 Gew.-% des Gesamtgewichts der Färbemittelzusammensetzung ausmachen.

13. Zusammensetzung nach einem der vorhergehenden Ansprüche, **dadurch gekennzeichnet, dass** die Additionssalze mit einer Säure unter Hydrochloriden, Hydrobromiden, Sulfaten, Tartraten, Lactaten und Acetaten ausgewählt sind.

14. Verfahren zum Färben von Keratinfasern und insbesondere menschlichen Keratinfasern, wie dem Haar, **dadurch gekennzeichnet, dass** auf die Fasern mindestens eine in einem der Ansprüche 1 bis 13 definierte Färbemittelzusammensetzung aufgebracht wird, wobei die Farbe bei saurem, neutralem oder alkalischem pH-Wert mit einem Oxidationsmittel entwickelt wird, das unmittelbar bei der Anwendung zu der Färbemittelzusammensetzung gegeben wird oder das in einer oxidierenden Zusammensetzung vorliegt, die gleichzeitig oder getrennt davon anschließend aufgebracht wird.

15. Verfahren nach Anspruch 14, **dadurch gekennzeichnet, dass** das Oxidationsmittel, das in der oxidierenden Zusammensetzung vorliegt, unter Wasserstoffperoxid, Harnstoffperoxid, Alkalimetallbromaten und Salzen von Persäuren, wie Perboraten und Persulfaten, ausgewählt ist.

16. Vorrichtung mit mehreren Abteilungen oder "Kit" zum Färben mit mehreren Abteilungen, wobei eine erste Abteilung eine in einem der Ansprüche 1 bis 13 definierte Färbemittelzusammensetzung und eine zweite Abteilung eine oxidierende Zusammensetzung enthält.

17. 4-Amino-2,2-dimethyl-2,3-dihydro-benzofuran-7-ol,
7-Amino-1-methyl-*1H*-indol-4-ol
und die Additionssalze dieser Verbindungen mit einer Säure.

18. Verwendung der in Anspruch 1 definierten p-Aminophenole der Formel (I) als Oxidationsbasen in Zusammensetzungen zum oxidativen Färben von Keratinfasern.
